# EUROPÄISCHE PATENTSCHRIFT

(11) **EP 0 989 404 B1**
(45) Veröffentlichungstag und Bekanntmachung des Hinweises auf die Patenterteilung: **25.06.2008**
(21) Anmeldenummer: 99118704.8
(22) Anmeldetag: 22.09.1999
(51) Int. Cl.: G01N 33/18

(54) **Wasser- und Abwasseranalysevorrichtung**
Analyser for water and waste water
Dispositif d'analyse d'eau et des eaux usées

(30) Priorität: 23.09.1998 DE 19843750; 10.11.1998 DE 29820171 U
(43) Veröffentlichungstag der Anmeldung: 29.03.2000
(73) Patentinhaber: WTW Wissenschaftlich-Technische Werkstätten GmbH & Co. KG, 82362 Weilheim (DE)
(72) Erfinder: Schwab, Ulrich, 82166 Graefelfing (DE)
(74) Vertreter: Zipse Habersack Kritzenberger

(56) Entgegenhaltungen:
- EP-B1- 0 989 405
- DE-A1- 4 114 959
- DE-A1- 19 819 857
- DE-U1- 29 701 652
- GB-A- 1 491 068
- US-A- 4 404 284
- US-A- 4 763 537
- US-A- 5 162 077
- US-A- 5 672 319

## Beschreibung

Die vorliegende Erfindung betrifft eine Wasser- und Abwasseranalysevorrichtung mit einer Probenahmeanordnung zur Bestimmung wenigstens eines Wasser- oder Abwasserparameters gemäß dem Oberbegriff des Anspruchs 1.

Eine derartige gattungsgemäße Anordnung, die für eine Vorortmessung geeignet ist, ist aus der US 5,672,319 bekannt. Bei dieser bekannten Vorrichtung ist es jedoch möglich, dass die Exaktheit der Werte mit zunehmender Nutzungsdauer leidet.

Der Erfindung liegt daher die Aufgabe zugrunde, eine Wasser- und Abwasseranalysevorrichtung zu schaffen, die exakte Werte liefert und verschmutzungsunempfindlich ist.

Diese Aufgabe wird gelöst durch eine Wasser- und Abwasseranalysevorrichtung gemäß Anspruch 1, welche die Reinigung einer Filtermembran einer Probenanordnung in einer Wasser- und Abwasseranalysevorrichtung und damit einen sicheren Betrieb auch in Wasser- und Klärbecken ermöglicht und keine separate Frischwasserversorgung benötigt. Vorteilhafte Weiterbildungen der Erfindung sind Gegenstand der Unteransprüche.

Erfindungsgemäß wird zur Gewinnung einer Probe das zu analysierende Wasser oder Abwasser durch eine Filtermembran geleitet und erst dann dem Analysegerät zugeführt. Weiterhin ist eine Rückspüleinrichtung vorgesehen, die die Membran vom Filtratraum aus mit Frischwasser oder Gas beaufschlagt. Hierdurch werden Verschmutzungen der Membran in den Probenraum gedrückt. Eine autarke und frischwasser- wie auch gasunabhängige Vorrichtung wird dadurch erzielt, dass während der normalen Tätigkeit das Filtrat aus dem Filtratraum in einem Permeatraum gespeichert wird und die Rückspüleinrichtung beim Rückspülen der Membran auf das filtrierte Wasser aus dem Permeatraum zugreift. Hier ist eine effiziente Reinigung der Membran ohne Frischwasser oder Gas möglich.

Um die Membran sauber zu halten, kann die gesamte Probenahmeanordnung im Klärbecken beispielsweise in einem Bereich hoher Turbulenzen oder starker Gaszufuhr angeordnet sein.

Im normalen Filter- und Analysebetrieb kann das Filtrat ständig in den Permeatraum gepumpt oder geführt werden, wobei der Ablauf aus dem Permeatraum möglichst als Überlauf ausgebildet ist, der wieder in den Probenraum bzw. das Becken zurückgeführt wird. In diesem Fall wird dem Permeatraum ständig frisches Permeat zugeführt, so daß die Bildung organischer Materie in dem Permeatraum unterbunden wird.

Eine Säuberung der Membran wird weiter unterstützt, wenn die Wasser- und Abwasseranalysevorrichtung eine separate Gaszufuhr enthält, die das Filtermedium mit Gas umspült. In diesem Falle kann die Probenahmeanordnung auch in einem weniger turbulenten oder gasdurchspülten Bereich eines Wasser- oder Klärbeckens angeordnet werden. Vorzugsweise ist die Gaszufuhreinrichtung dann unterhalb der Membran vorgesehen.

Die Wasser- und Abwasseranalysevorrichtung kann z.B. einen separaten Probenraum aufweisen, der über eine Zuleitung von einem Wasser- oder Klärbecken gespeist wird. Die Membran kann jedoch auch direkt in einem Wasser- oder Klärbecken angeordnet sein, so daß dieses den Probenraum bildet.

Vorzugsweise sind Zu- und Ablauf in dem Permeatraum so ausgebildet, daß dort im Normalbetrieb eine lineare Strömung aufrechterhalten wird. Die Rückspüleinrichtung kann zusätzlich mit einer Reinigungsmittelzufuhr, zum Beispiel einem Reinigungsmittelbehälter verbunden sein, durch den auch eine Reinigungsflüssigkeit dem Filtratraum zugeführt werden kann. Vorzugsweise ist die Rückspüleinrichtung durch eine Pumpe gebildet, die einen geeigneten Überdruck in dem Filtratraum erzeugen kann, um Verschmutzungen, insbesondere organischer Natur, in der Membran in Richtung auf den Probenraum auszuspülen.

In einer weiteren sehr verschmutzungsunanfälligen Ausbildungsform der Erfindung ist im Bereich der Membran, vorzugsweise im Filtratraum, ein Ultraschallerzeugungsgerät angeordnet, das das Wasser im Bereich der Membran in entsprechende Schwingung versetzt und damit die Membran vor Verstopfung freihält. Dieses Ultraschallerzeugungsgerät kann beispielsweise ein Piezoelement sein. Das Ultraschallerzeugungsgerät wird vorzugsweise von der Steuerung der Analysevorrichtung gesteuert.

Selbstverständlich läßt sich die Rückspüleinrichtung wahlweise mit der Ultraschallerzeugung und/oder der separaten Gaszufuhr kombinieren, um auf diese Weise noch effizienter die Verschmutzung der Membran in dem durch organische Materialien verschmutzten Klärbecken zu unterbinden.

In einer sehr kompakten und anwenderfreundlichen Ausbildung der Erfindung ist das Analysegerät und die Probenahmeanordnung mit der Filtermembran integriert in einem Gehäuse ausgebildet, welches sich in ein Wasser- oder Klärbecken absenken läßt. Hier ist die gesamte Wasser- und Abwasseranalysevorrichtung direkt am Meßort untergebracht und führt dazu, daß weder ein separater Platz am Beckenrand oder in einem Gebäude der Kläranlage für das Abwasseranalysegerät noch eine separate Probenahmeanordnung bereitgestellt werden muß. Dadurch, daß die gesamte Probenahmeanordnung mit Probenraum, Filtratraum, Analysegerät, Rückspüleinrichtung und gegebenenfalls einer Gaszufuhreinrichtung und einem Ultraschallerzeugungsgerät integriert ausgebildet sind, benötigt man lediglich ein verschmutzungsunempfindliches Tauchgehäuse, welches ohne den Betrieb zu stören, in ein Wasser- oder Klärbecken abgesenkt werden kann. Von diesem Gerät kann eine Leitung nach außen führen, auf der direkt ein Signal für den gewünschten Wasser- oder Abwasserparameter, zum Beispiel Nitrat- oder Phosphatwert, Nitrit- oder Ammoniakwert abrufbar ist.

Es ist selbstverständlich auch möglich, die integrierte Anordnung mit einer Sende/Empfangsanlage auszustatten, über welche die Meßergebnisse drahtlos an einen Empfänger in einem Gebäude der Kläranlage, zum Beispiel der zentralen Prozeßsteuerung gesandt werden können. Die integrierte Vorrichtung kann somit auch Steuerparameter von einer externen Steuerung erhalten. Im Wege der drahtlosen Übertragung wäre es auch möglich, den Betriebszustand der Wasser- und Abwasseranalysevorrichtung zu überprüfen wie zum Beispiel Batteriestatus, Chemikalien und Reinigungsmittelstatus und Betriebswerte von Pumpen, um Rückschlüsse auf den Druckabfall an der Filtermembran zu erhalten.

Das Analysegerät muß jedoch nicht bereits die fertigen Werte liefern, sondern lediglich Werte, die von einer Auswerteeinrichtung zur Bestimmung der Meßparameter interpretiert werden können. Vorzugsweise ist jedoch auch die Auswerteeinrichtung gleich in dem integrierten Gerät mit angeordnet.

Bei der integrierten Tauchanordnung ist vorzugsweise der Probenraum durch ein Sieb oder eine perforierte Außenwand des Gehäuses gebildet. Auf diese Weise wird das Eindringen grober Schmutzpartikel in den Probenraum unterbunden. In dem Probenraum ist dann die Filtermembran vorgesehen, die vorzugsweise von der Gaszufuhreinrichtung umspült wird, dessen Gaszufuhröffnung im unteren Bereich des Probenraums angeordnet ist. Die hierdurch hervorgerufenen Turbulenzen reinigen die Membranoberfläche. Alternativ dazu ist es jedoch selbstverständlich auch möglich, die Filtermembran direkt mit dem umgebenden Medium des Wasser- oder Klärbeckens in Kontakt treten zu lassen, wodurch die Filtermembran quasi eine Außenwand des Gehäuses bildet.

Vorzugsweise ist die Tauchsonde über einen Positionierstab in dem Wasser- oder Klärbecken gehalten. Der Positionierstab ragt vorzugsweise aus dem Wasser heraus und kann zum Beispiel Solarzellen zum Betrieb der elektrischen Komponenten der Analysevorrichtung an seiner Oberseite aufweisen. In dem Positionierstab können auch elektrische Leitungen oder Zufuhrleitungen für Reinigungsmittel und Reagenzien ausgebildet sein. Im Falle der Verwendung von Solarzellen ist vorzugsweise in der Tauchsonde ein Akkumulator vorgesehen, der über die Solarzellen aufgeladen wird und den notwendigen Strom für Pumpen, elektrische Ventile oder ein Ultraschallgerät bereitstellt.

Die Erfindung wird nachfolgend beispielsweise anhand der schematischen Zeichnung beschrieben. In dieser zeigen:

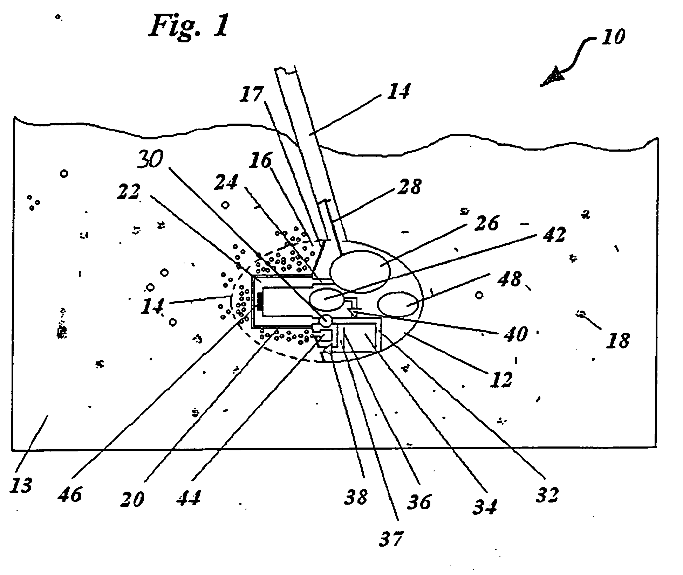

Fig. 1 eine integrierte Wasser- und Abwasseranalysevorrichtung in Form einer Tauchsonde.

Die Wasser- und Abwasseranalysevorrichtung 10 ist als Tauchsonde zur Absenkung in ein Wasser- oder Klärbecken 13 ausgebildet. Die Tauchsonde 10 hat ein im wesentlichen rotationssymmetrisches eiförmiges Gehäuse 12, das von einem Positionierstab 14 unterhalb der Wasseroberfläche gehalten wird. Die Rückseite des Gehäuses 12 wird durch eine durchlässige Wand 14 gebildet, die einen Probenraum 16 vom Klärbecken 13 abtrennt. Auf diese Weise gelangen Verschmutzungen 18 nicht in den Probenraum 16. Koaxial zur Rotationsachse des Gehäuses 12 ist in dem Probenraum 16 eine becherförmige zylindrische Filtermembran 20 angeordnet. Diese Filtermembran 20 kann entweder aus einem entsprechend steifen Material bestehen oder einen becherförmigen Träger aufweisen, der von einer dünnen Membran umgeben ist. Durch die Filtermembran 20 wird der Probenraum 16 von dem Filtratraum 22 abgetrennt. Eine Trennwand 17 trennt den Probenraum 16 von den übrigen Komponenten der Wasser- und Abwasseranalysevorrichtung 10 ab. Der Filtratraum 22 ist über eine Zuführung 24 mit einem Analysegerät 26 verbunden, das wiederum über eine elektrische Anschlußleitung 28 mit einer Meßdatenerfassung und/oder Energieversorgung verbunden sein kann. Die elektrische Datenübertragungs- und Versorgungsleitung 28 führt in dem Positionierstab 14 entlang aus dem Wasser- bzw. Klärbecken 12 heraus. Der Filtratraum 22 ist weiterhin über eine Pumpe 30 und eine Rückspülleitung 32 mit einem Permeatraum 34 verbunden, der über einen Überlauf 36, einen Rücklauf 37 und ein erstes Magnetventil 38 wieder mit dem Probenraum verbunden ist. Im normalen Filtrations- und Analysebetrieb wird ständig von der Pumpe 30 Filtrat über die Rückspülleitung 32 in den Permeatraum 34 gepumpt. Älteres Permeat läuft über den Überlauf 36, den Rücklauf 37 und das erste Magnetventil 38 in den Probenraum zurück. Alternativ könnte der Rücklauf auch direkt an einer Außenseite des Gerätes in das Klärwasser 12 münden. In die Rückspülleitung 32 mündet über ein zweites Magnetventil 40 der Ausgang eines Reinigungsmittelbehälters 42. Über das zweite Magnetventil 40 kann die Reinigungsmittelzufuhr aus dem Reinigungsmittelbehälter 42 geöffnet werden, um auf diese Weise den Filtratraum 22 und/oder den Permeatraum 34 zu reinigen.

Wenn sich die Filtermembran 20 im Verlauf der normalen Analysetätigkeit langsam zusetzt, kann die Pumpe 30 in Gegenrichtung geschaltet und das erste Magnetventil 38 geschlossen werden. In diesem Fall wird sauberes Filtrat aus dem Permeatraum 34 in den Filtratraum 22 gepumpt, wodurch die Filtermembran 22 unter Druck rückgespült wird. Hierbei kann über eine entsprechende Ansteuerung des zweiten Magnetventils 40 auch ein Reinigungsmittel aus dem Reinigungsmittelbehälter 42 zugeführt werden. Auf diese Weise läßt sich die Filtermembran sehr gut reinigen.

Die Reinigung der Membran 22 kann zusätzlich auch durch eine Gaszufuhreinrichtung 44 realisiert werden, die unterhalb der Filtermembran 20 in den Probenraum 16 mündet und Gasblasen bzw. Gas in den Probenraum einspritzt, wodurch das Filtermedium durch die Gasblasen und die entstehenden Turbulenzen mechanisch gereinigt wird.

Zur zusätzlichen oder alternativen zusätzlichen Reinigung der Membran 20 ist ein Ultraschallgerät 46 vorgesehen, welches im vorliegenden Fall in dem Filtratraum 22 angeordnet ist. Durch die Ultraschallschwingungen wird die Filtermembran in eine Schwingung versetzt, wodurch in den Poren befindliche Verunreinigungen schnell und wirkungsvoll gelöst werden. Die genannten zusätzlichen Reinigungsvorrichtungen können alternativ oder auch in Kombination miteinander betrieben werden, wodurch sich auch in stark verschmutzten Klärbecken eine wirkungsvolle Analyse durchführen läßt.

In dem Gehäuse 12 ist weiterhin ein Behälter 48 für Reagenzien bzw. Chemikalien vorgesehen, die zum Betrieb des Analysegerätes 26 notwendig sind.

Kein Bestandteil der Erfindung ist ein Verfahren zur Reinigung einer Filtermembran (20) einer Probenahmeanordnung in einer Wasser- und Abwasseranalysevorrichtung, die ein Analysegerät (26) zur Bestimmung wenigstens eines Wasser- oder Abwasserparameters aufweist, in dem:
- eine Probe über eine Membran (20) von einem Probenraum (16) in einen Filtratraum (22) geführt und die Probe dem Analysegerät (26) zugeführt wird,
- das Filtrat vom Filtratraum (22) in einen Permeatraum (34) geleitet wird, und
- während eines Rückspülvorgangs die Membran (20) vom Filtratraum (22) aus mittels einer Rückspüleinrichtung (30 - 38) rückgespült wird, wobei das zum Rückspülen erforderliche Wasser aus dem Permeatraum (34) entnommen wird.

Vorzugsweise wird hier dem Permeatraum (34) ständig Permeat aus dem Filtratraum (22) zugeführt, und das dem Permeatraum (34) überschüssig zugeführte Permeat als Überlauf in den Probenraum (16) zurückgeführt.

Vorzugsweise weist der Permeatraum in seinem unteren Bereich eine Zuleitung vom Filtratraum und in seinem oberen Bereich eine Rückleitung zum Probenraum auf oder vice versa, wobei insbesondere die Rückleitung (37) an einem Ende in einen Überlauf (36) des Permeatraums (34) mündet. Es ist darüber hinaus vorteilhaft, wenn die Rückspüleinrichtung (30 - 38) eine Pumpe (30) und steuerbare Elektroventile (38,40) umfaßt, um Wasser aus dem Permeatraum (34) und/oder aus der Reinigungsmittelzufuhr (42) in den Filtratraum (22) zu pumpen. Wenn das Ultraschallerzeugungsgerät (46) ein Piezoelement aufweist und von einer Steuerung der Analysevorrichtung (10) steuerbar ist, läßt sich eine sehr effektive Säuberung oder Sauberhaltung der Membran erreichen.

## Patentansprüche

1. Wasser- und Abwasseranalysevorrichtung mit einer Probenahmeanordnung umfassend
- ein Analysegerät (26) zur Bestimmung wenigstens eines Wasser- oder Abwasserparameters,
- eine Membran (20), die einen Probenraum (16) von einem Filtratraum (22) trennt, und
eine Leitung (24), die den Filtratraum (22) mit dem Analysegerät (26) verbindet,
**gekennzeichnet durch** einen Permeatraum (34), in dem während der normalen Tätigkeit der Vorrichtung das Filtrat aus dem Filtratraum (22) gespeichert wird, wobei
der Filtratraum (22) mit dem Permeatraum (34) verbunden und eine Rückspüleinrichtung (30 - 38) zum Rückspülen der Membran (20) vom Filtratraum (22) aus vorgesehen ist, und wobei die Rückspüleinrichtung (30 - 38) mit dem Permeatraum (34) verbunden ist, um das zum Rückspülen erforderliche Wasser aus dem Permeatraum (34) zu beziehen.

2. Vorrichtung nach Anspruch 1,
**dadurch gekennzeichnet,**
**daß** die Rückspüleinrichtung (30 - 38) oder der Permeatraum (34) mit einer Reinigungsmittelzufuhr (40,42) verbindbar ist.

3. Vorrichtung nach einem der vorhergehenden Ansprüche,
**dadurch gekennzeichnet,**
**daß** eine Gaszufuhreinrichtung (44) zum Umspülen der Membran (22) vorgesehen ist.

4. Vorrichtung nach Anspruch 3,
**dadurch gekennzeichnet,**
**daß** die Gaszufuhreinrichtung (44) im Probenraum (16) unterhalb der Membran (20) angeordnet ist.

5. Vorrichtung nach einem der vorhergehenden Ansprüche,
**dadurch gekennzeichnet,**
**daß** im Bereich der Membran (20) ein Ultraschallerzeugungsgerät (46), insbesondere im Filtratraum (22) vorgesehen ist.

6. Vorrichtung nach einem der vorhergehenden Ansprüche,
**dadurch gekennzeichnet,**
**daß** die Probenahmeanordnung mit dem Analysegerät (26) in einem Gehäuse (12) integriert ist, welches in ein Wasser- oder Klärbecken (13) absenkbar ist.

7. Vorrichtung nach Anspruch 6,
**dadurch gekennzeichnet,**
**daß** in dem Gehäuse (12) ein Behälter (48) für Referenzchemikalien und/oder der Permeatraum (34) zur Aufnahme des Filtrats vorgesehen ist.

8. Vorrichtung nach Anspruch 6 oder 7,
**dadurch gekennzeichnet,**
**daß** der Probenraum (16) über ein an dem Gehäuse (12) angeordnetes Sieb, Gitter oder eine perforierte Wand (14) gegen das Klär- oder Wasserbecken (13) abgegrenzt ist.

9. Vorrichtung nach einem der Ansprüche 6 bis 8,
**dadurch gekennzeichnet,**
**daß** das Gehäuse (12) und der Probenraum (16) im wesentlichen rotationssymmetrisch ausgebildet, und der Filtratraum (22) durch eine koaxial zum Gehäuse verlaufende zylindrische Membran (20) von dem Probenraum (16) abgetrennt ist.

## Claims

1. An apparatus for analyzing water and wastewater including a sampler, comprising:
- an analyzer (26) for determining at least one water or wastewater parameter;
- a membrane (20) separating a sample space (16) from a filtrate space (22); and
a conduit (24) connecting the filtrate space (22) to the analyzer (26);
**characterized by** a permeate space (34) in which during normal activitity of the apparatus the filtrate from the filtrate space (22) is stored, the filtrate space (22) being connected to the permeate space (34) and a backwasher (30 - 38) for backwashing the membrane (20) from the filtrate space (22) being provided, and the
backwasher (30 - 38) being connected to the permeate space (34) to obtain water needed for backwashing from the permeate space (34).

2. The apparatus as set forth in claim 1, **characterized in that** the backwasher (30 - 38) or permeate space (34) is connectable to a cleaning agent feed (40, 42).

3. The apparatus as set forth in any of the preceding claims, **characterized in that** a gas feeder (44) is provided for flushing the membrane (20).

4. The apparatus as set forth in claim 3 **characterized in that** the gas feeder (44) is arranged in the sample space (16) below the membrane (20).

5. The apparatus as set forth in any of the preceding claims, **characterized in that** an ultrasonic generator is provided in the region of the membrane (20), particularly in the filtrate space (22).

6. The apparatus as set forth in any of the preceding claims, **characterized in that** the sampler is integrated with the analyzer (26) in a housing (12) which is immersible in a water tank or clarifying tank (13).

7. The apparatus as set forth in claim 6, **characterized in that** the housing (12) comprises a tank (48) for reference chemicals and/or the permeate space (34) for accommodating the filtrate.

8. The apparatus as set forth in claim 6 or 7, **characterized in that** the sample space (16) is defined from a clarifying tank or water tank (13) by a strainer, grille or a perforated wall (14) arranged at the housing (12).

9. The apparatus as set forth in any of the claima 6 to 8, **characterized in that** the the housing and the sample space (16) are configured substantially rotationally-symmetrical, and the filtrate space (22) is separated from the sample space (16) by a cylindrical membrane (20) running coaxial to the housing.

## Revendications

1. Dispositif d'analyse d'eau et des eaux usées munie d'une installation de prélèvement d'échantillons comportant :
- un dispositif d'analyse (26) prévu pour définir au moins un paramètre de l'eau ou des eaux usées,
- une membrane (20) formant une séparation entre une chambre à échantillons (16) et une chambre à filtrat (22), et
- une conduite (24) reliant la chambre à filtrat (22) au dispositif d'analyse (26),
**caractérisé par** une chambre de perméat (34), dans laquelle le filtrat provenant de la chambre à filtrat (22) est stocké pendant l'activité normale du dispositif, la chambre à filtrat (22) étant reliée à la chambre de perméat (34) et un dispositif de lavage à contre-courant (30 - 38) étant prévu pour le rétro-rinçage de la membrane (20) à partir de la chambre à filtrat (22), et le dispositif de lavage à contre-courant (30 - 38) étant relié à la chambre de perméat (34), afin qu'il s'approvisionne en eau, provenant de la chambre de perméat (34), nécessaire au rétro-rinçage.

2. Dispositif selon la revendication 1,
**caractérisé par le fait que** le dispositif de lavage à contre-courant (30 - 38) ou la chambre de perméat (34) peut être relié(e) à une alimentation en produit de lavage (40, 42).

3. Dispositif selon l'une des revendications précédentes,
**caractérisé par le fait qu'**un dispositif d'alimentation en gaz (44) est destiné au rinçage de la membrane (22).

4. Dispositif selon la revendication 3,
**caractérisé par le fait que** le dispositif d'alimentation en gaz (44) est disposé dans la chambre à échantillons (16) en dessous de la membrane (20).

5. Dispositif selon l'une des revendications précédentes,
**caractérisé par le fait qu'**un appareil à ultrasons (46) est prévu au niveau de la membrane (20), en particulier dans la chambre de filtrat (22).

6. Dispositif selon l'une des revendications précédentes,
**caractérisé par le fait que** l'installation de prélèvement d'échantillons dotée du dispositif d'analyse (26) est intégrée dans un boîtier (12) pouvant être abaissé dans un bassin d'eau ou de décantation (13).

7. Dispositif selon la revendication 6,
**caractérisé par le fait qu'**un récipient (48) pour les produits chimiques de référence et/ou la chambre de perméat (34) est prévu(e) dans le boîtier (12) pour recevoir le filtrat.

8. Dispositif selon la revendication 6 ou 7,
**caractérisé par le fait qu'**un tamis, une grille ou une paroi perforée (14) disposé(e) sur le boîtier (12) séparant la chambre à échantillons (16) du bassin d'eau ou de décantation (13).

9. Dispositif selon l'une quelconque des revendications 6 à 8,
**caractérisé par le fait que** le boîtier (12) et la chambre à échantillons (16) sont essentiellement formés en symétrie de rotation, et que la chambre de filtrat (22) est séparée de la chambre à échantillons (16) par une membrane cylindrique (20) s'étendant de manière coaxiale par rapport au boîtier.
